# EUROPEAN PATENT APPLICATION

(11) **EP 1 925 670 A1**
(43) Date of publication of application: **28.05.2008**
(21) Application number: 06775269.1
(22) Date of filing: 02.08.2006
(51) Int. Cl.: C12N 15/70, C12N 15/72, C12N 15/68, C12N 9/06, C12N 9/14, C12P 21/02, C12P 35/06

(54) **ESCHERICHIA COLI EXPRESSION VECTOR AND THE USES THEREOF**

(30) Priority: 08.08.2005 CN 200510089966
(71) Applicant: Bioright Worldwide Company Limited, Road Town, Tortola (VG)
(72) Inventor: WANG, Jun, Hong Kong (CN); TSANG, Waikei, Hong Kong (CN); YAP, Hongkin, Hong Kong (CN); LIU, Zhaoming, Hong Kong (CN); SIU, Yaulung, Hong Kong (CN); TSANG, Supyin, Hong Kong (CN)
(74) Representative: Eisenführ, Speiser & Partner
(86) International application number: PCT/CN2006/001939
(87) International publication number: WO 2007/016860

(57) **Abstract**

The present invention discloses *Escherichia coli* expression vector, as well as a method for steadily, high-efficiently expressing a required target protein in *E. coli* using the expression vector. The expression vector is constructed from vector pRSET-A, wherein ampicillin resistance gene in said vector pRSET-A is replaced by kanamycin resistance gene. The expression vector can be used to high-efficiently express enzymes required for the production of 7-aminocephalosporanic acid, meanwhile it does not produce β-lactamase by which the productivity of 7-aminocephalosporanic acid is reduced. The present invention also discloses two expression vectors, i.e., one expression vector pHS-GHA whose gene product is D-amino acid oxidase mutant GHA, and another expression vector pT7-kan-ACY whose gene product is glutaryl-7-aminocephalosporanic acid acylase of *Pseudomonas* sp. SE83.

## Description

### Field of the Invention

The present invention relates to biotechnology, and more specifically, relates to the preparation of an *Escherichia coli* expression vector, which does not produce β-lactamase and can be used to high-efficiently express enzymes required for the production of 7-aminocephalosporanic acid.

### Background of the Invention

Enzymatic production of 7-aminocephalosporanic acid (7-ACA) is conducted in two steps: (1) cephalosporin C (CPC) is first oxidized by D-amino acid oxidase to glutaryl-7-ACA; (2) glutaryl-7-ACA is in turn cleaved by glutaryl-7-ACA acylase to 7-ACA. At present, most of the above enzymes are produced from expression vectors containing ampicillin resistance gene as selectable marker. However, its gene product, β-lactamase, besides degrading ampicillin, also degrades β-lactams such as CPC, glutaryl-7-ACA and 7-ACA. Therefore, in a fermentation system using expression vectors containing the ampicillin resistance gene, the 7-ACA yield is low due to the presence of high level of β-lactamase. In addition, the above-mentioned expression vectors in *E. coli* is low in stability and is easily lost during fermentation, thus affecting production of target proteins. Current reports on the production level of D-amino acid oxidase is low, about 2,300 U/L fermentation medium (Pollegioni, L. et al., 1997, J. Biotechnol. 58, 115-123) and 800 U/L fermentation medium (Molla, G. et al., 1998, Protein Exp. Purif. 14, 289-294). The production level of glutaryl-7-ACA acylase production level is also low, about 129-2,500 U/L fermentation medium (Ishiye, M. and Niwa, M., 1992, Biochim. Biophys. Acta 1132, 233-239; Yang, Y. L. et al., 2001, CN1301813A; Xu, G. and Zhu, M. 2003, CN1428424A). Consequently the current enzymatic production of 7-ACA are high in cost and low in yield.

### Summary of the Invention

One object of the invention is to provide a stable and efficient *E. coli* expression vector, which does not produce β-lactamase. It can be used to high-efficiently express enzymes required for production of 7-ACA, thus increasing the conversion rate of 7-ACA and reducing the cost. Another object of the invention is to provide a method to use said vector to high-efficiently express target proteins in *E. coli.*

To achieve the above objects, the invention provides an *E. coli* expression vector which contains a promoter to control the expression of target proteins. Said vector is constructed from vector pRSET-A, wherein the ampicillin resistance gene is replaced by a kanamycin resistance gene.

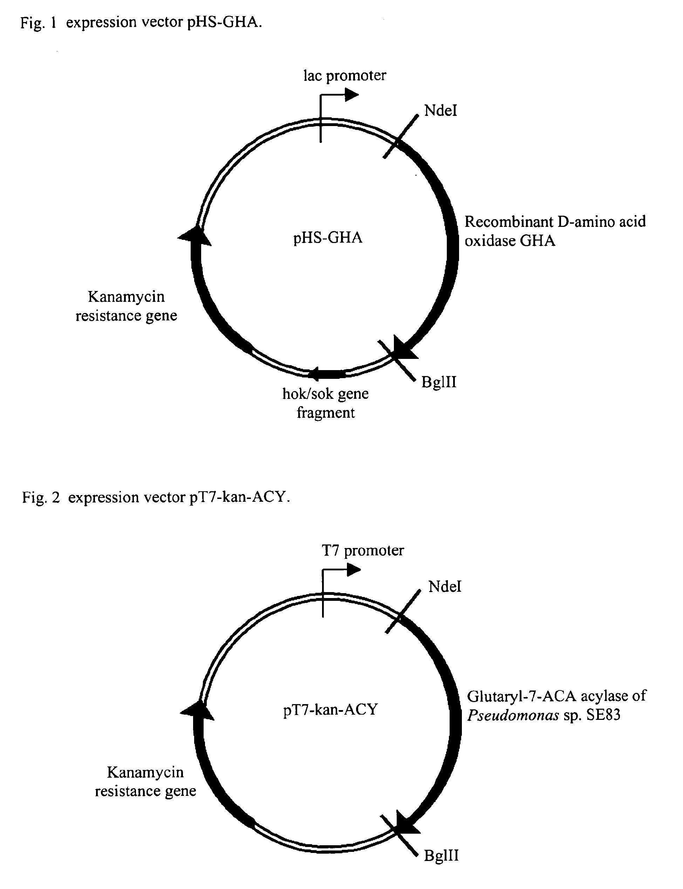

Preferably, the promoter in said expression vector is lac promoter and said expression vector contains hok/sok genes, more preferably also contains ribosome binding sequence AGAAGGA to facilitate the translation of proteins. Preferably, the target protein is D-amino acid oxidase, more preferably is *Trigonopsis variabilis* D-amino acid oxidase mutants. More preferably, the invention provides an expression vector pHS-GHA (Fig. 1), with the nucleic acid sequence as Sequence 1 in Sequence Listing.

The above mentioned hok/sok genes encode hok RNA (target RNA) and sok RNA (antisense RNA). The gene product of hok destructs cell membrane and causes cell death. The sok RNA inhibits the translation of hok RNA, thus preventing production of hok gene product. *E. coli* that carries plasmid containing hok/sok genes is viable. On the contrary, *E. coli* will not survive if the plasmid is lost as hok RNA is more stable than sok RNA (Pecota, D. C. et al., 1997, Appl. Environ. Microbiol. 63, 1917-1924).

In another aspect of the expression vector provided in the invention, the target protein is glutaryl-7-ACA acylase gene, preferably from *Pseudomonas* sp. SE83. More preferably, the expression vector provided in the invention is pT7-kan-ACY (Fig. 2), with the nucleic acid sequence as Sequence 2 in Sequence Listing.

In another aspect, the invention provides a method to express target proteins in *E. coli,* said E. *coli* carries a plasmid that contains a target protein gene, controlled by a promoter. The expression vector is constructed from vector pRSET-A, wherein the ampicillin resistance gene is replaced by a kanamycin resistance gene. Said target proteins preferably are D-amino acid oxidase or glutaryl-7-ACA acylase. Said E. *coli* is cultivated in medium containing 50-100µg/mL kanamycin.

### Brief Description of the Drawings

Fig. 1 shows expression vector pHS-GHA.
Fig. 2 shows expression vector pT7-kan-ACY.
Fig. 3 shows expression vector pRSET-lac-GI-hok/sok-kan.
Fig. 4 shows the HPLC chromatogram of the degradation of CPC/glutaryl-7-ACA of D-amino acid oxidase GHA from BL-HS-GHA.
Fig. 5 shows the HPLC chromatogram of the degradation of CPC/glutaryl-7-ACA of D-amino acid oxidase GHA from BL-T7K-GHA.
Fig. 6 shows the HPLC chromatogram of the degradation of CPC/glutaryl-7-ACA of D-amino acid oxidase GHA from BL-T7A-GHA.
Fig. 7 shows the HPLC chromatogram of the degradation of glutaryl-7-ACA/7-ACA of glutaryl-7-ACA acylase of *Pseudomonas* sp. SE83 from BL-T7K-ACY.
Fig. 8 shows the HPLC chromatogram of the degradation of glutaryl-7-ACA/7-ACA of glutaryl-7-ACA acylase of *Pseudomonas* sp. SE83 from BL-T7A-ACY.

### Example 1

### Construction of vector pRSET-kan

The following primers were synthesized based on the sequence of pRSET-A:
VET-F
   5'-CTGTCAGACCAAGTTTACTCATATATACTTTAG-3'
VET-R
   5'-ACTCTTCCTTTTTCAATATTATTGAAGC-3'

The following primers were synthesized based on the sequence of pET-28b:
KAN-F
   5'-ATGAGCCATATTCAACGGGAAAC-3'
KAN-R
   5'-TTAGAAAAACTCATCGAGCATCAAATG-3'
PCR mixture for amplifying pRSET-A fragment devoid of ampicillin resistance gene contained: 50ng pRSET-A (Invitrogen), 0.4µM VET-F, 0.4µM VET-R, 50µM dATP, 50µM dTTP, 50µM dCTP, 50µM dGTP, 20mM Tris-HCI (pH8.8), 10mM KCI, 10mM (NH₄)₂SO₄, 2mM MgSO₄, 0.1% Triton X-100, 2.5U Pfu DNA polymerase (Promega). The volume of the mixture was made up to 50µL with sterile deionized water.

PCR profile was as follows:
94°C,1min 35 cycles
94°C, 5 min →50°C, 1 min →→→→→ 72°C, 10 min
72°C, 4 min

PCR mixture for amplifying kanamycin resistance gene from plasmid pET-28b contained: 50ng pET-28b (Novagen), 0.4µM KAN-F, 0.4µM KAN-R, 50µM dATP, 50µM dTTP, 50µM dCTP, 50µM dGTP, 20mM Tris-HCl (pH8.8), 10mM KCI, 10mM (NH₄)₂SO₄, 2mM MgSO₄, 0.1% Triton X-100, 2.5U Pfu DNA polymerase (Promega). The volume of the mixture was made up to 50µL with sterile deionized water.

PCR profile was as follows:
94°C, 1 min 35 cycles
94°C, 5 min →50°C, 1 min →→→→→ 72°C, 10 min
72°C, 4 min

The two PCR products (pRSET-A fragment devoid of ampicillin resistance gene, 2,036bp in size; the kanamycin resistance gene, 816bp in size) were resolved in 0.8% agarose, purified and ligated to generate plasmid pRSET-kan. The plasmid was used to transform competent E. *coli* BL21(DE3)pLysS (Novagen), spread onto LB (1% sodium chloride, 1% peptone, 0.5% yeast extract) agar containing 50µg/mL kanamycin and incubated at 37°C overnight. Plasmid was extracted in accordance with Molecular Cloning-A Laboratory Manual (Sambrook, J. et al., 1989, CSHL Press).

### Example 2

### Construction of vector pRSET-lac-kan

The following primers were synthesized based on the sequence of pGEMT-Easy (Promega):
RBS-NdeI
   5'-CATATGTATATCTCCTTCTTGTGTGAAATTG-3'
(NdeI restriction site is underlined and ribosome binding site is marked by broken underline);
RBS-AlwNI
   5'-CAGTGGCTGCTGCCAGTGGCGATAAGTC-3'
(AlwNI restriction site is underlined).
PCR was performed using pGEMT-Easy (Promega) as template to generate a 755bp PCR product. PCR mixture contained 50ng pGEMT-Easy (Promega), 0.4µM RBS-NdeI, 0.4µM RBS-AlwNI, 50µM dATP, 50µM dTTP, 50µM dCTP, 50µM dGTP, 20mM Tris-HCl (pH8.8), 10mM KCI, 10mM (NH₄)₂SO₄, 2mM MgSO₄, 0.1% Triton X-100, 2.5U Pfu DNA polymerase (Promega). The volume of the mixture was made up to 50µL with sterile deionized water.

PCR profile was as follows:
94°C, 1 min 35 cycles
94°C, 5 min →50°C, 1 min →→→→→ 72°C, 10 min
72°C, 4 min

The PCR product (755bp) contains NdeI restriction site and ribosome binding site at the 5' end and AlwNI restriction site at the 3' end. The PCR product was resolved in 0.8% agarose, purified, digested by NdeI and AlwNI, and ligated with NdeI/AlwNI restricted pRSETA (Invitrogen) to generate pRSET-lac. The plasmid was used to transform competent E. *coli* BL21(DE3)pLysS (Novagen), spread onto LB (1% sodium chloride, 1% peptone, 0.5% yeast extract) agar containing 100µg/mL ampicillin and incubated at 37°C overnight. Plasmid was extracted in accordance with Molecular Cloning-A Laboratory Manual (Sambrook, J. et al., 1989, CSHL Press).

Vectors pRSET-1ac and pRSET-kan were cut with AlwNI and EcoRI and resolved in 0.8% agarose, purified and ligated, generating pRSET-lac-kan. The plasmid was used to transform competent *E. coli* BL21(DE3)pLysS (Novagen), spread onto LB agar containing 50µg/mL kanamycin and incubated at 37°C overnight. Plasmid was extracted in accordance with Molecular Cloning-A Laboratory Manual (Sambrook, J. et al., 1989, CSHL Press).

### Example 3

### Construction of vector pGEMT-Easy-GI

The following primers were synthesized based on the sequence of known *Thermoanaerobacterium saccharolyticum* glucose isomerase gene (GenBank L09699).
GI-NdeI
   5'-CATATGAATAAATATTTTGAGAACGTATCTAAAATA-3'
(NdeI restriction site is underlined);
   GI-EcoRI
5'-GATATCTTAAGGCGCGCCTTATTCTGCAAAC-3'
   (EcoRI restriction site is underlined and AscI restriction site is double underlined).

PCR was performed using *Thermoanaerobacterium saccharolyticum* (purchased from ATCC, USA) DNA as template to generate a 1,336bp PCR product. PCR mixture contained 50ng *T. saccharolyticum* DNA, 0.4µM GI-NdeI, 0.4µM GI-EcoRI, 50µM dATP, 50µM dTTP, 50µM dCTP, 50µM dGTP, 20mM Tris-HCl (pH8.8), 10mM KCI, 10mM (NH₄)₂SO₄, 2mM MgSO₄, 0.1% Triton X-100, 2.5U Platinum Taq High Fidelity DNA polymerase (Invitrogen). The volume of the mixture was made up to 50µL with sterile deionized water.

PCR profile was as follows:
94°C, 1 min 35 cycles
95°e, 5 min →50°C, 1 min →→→→→ 72°C, 10 min
72°C, 3 min

The PCR product (1,336bp) contains NdeI restriction site at the 5' end and EcoRI restriction site at the 3' end. The PCR product was resolved in 0.8% agarose, purified and ligated to pGEMT-Easy (Promega) by TA cloning, generating pGEMT-Easy-GI. The plasmid was used to transform competent E. *coli* DH5α (Invitrogen), spread onto LB agar containing 100µg/mL ampicillin and incubated at 37°C overnight. Plasmid was extracted in accordance with Molecular Cloning-A Laboratory Manual (Sambrook, J. et al., 1989, CSHL Press).

### Example 4

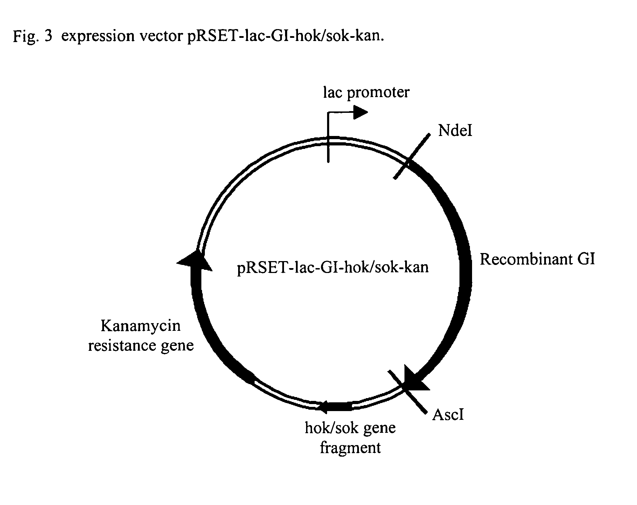

### Construction of vector pRSET-lac-GI-hok/sok-kan (Fig. 3)

Vector pGEMT-Easy-GI was cut by NdeI and EcoRI and resolved in 0.8% agarose, purified and ligated to Ndel/EcoRl-digested pRSET-lac-kan, generating pRSET-lac-GI-kan. The plasmid was used to transform competent E. *coli* BL21(DE3)pLysS (Novagen), spread onto LB agar containing 50µg/mL kanamycin and incubated at 37°C overnight. Plasmid was extracted in accordance with Molecular Cloning-A Laboratory Manual (Sambrook, J. et al., 1989, CSHL Press).

Ten primers were synthesized based on known hok/sok gene sequence (GenBank X05813) (Table 1). PCR gene assembly was performed as described by Kikuchi, M. et al., 1999, Gene 236:159-167, with modifications. PCR mixture contained 20ng each primer, 50µM dATP, 50µM dTTP, 50µM dCTP, 50µM dGTP, 20mM Tris-HCl (pH8.8), 10mM KCI, 10mM (NH₄)₂SO₄, 2mM MgSO₄, 0.1% Triton X-100, 2.5U Pfu DNA polymerase (Promega). The volume of the mixture was made up to 50µL with sterile deionized water.

PCR profile was as follows:
94°C, 1.5 min 30 cycles
95°C, 4 min →50°C, 1.5 min →→→→→ 72°C, 10 min
72°C, 5 min

The PCR product (580bp) contains AscI restriction site at the 5' end and EcoRI restriction site at the 3' end. The PCR product was resolved in 0.8% agarose, cut with AscI and EcoRI and ligated to AscI/EcoRI-digested pRSET-lac-GI-kan, generating pRSET-lac-GI-hok/sok-kan. The plasmid was used to transform competent E. *coli* BL21(DE3)pLysS (Novagen), spread onto LB agar containing 50µg/mL kanamycin and incubated at 37°C overnight. Plasmid was extracted in accordance with Molecular Cloning-A Laboratory Manual (Sambrook, J. et al., 1989, CSHL Press) and sequenced as in Sequence 3 in the Sequence Listing. Nucleotide variations were observed in a number of positions: 1368 (C→G); 1513 (missed a T); 1804(A→T); 1826(C→T); 2479(G→T); 2555(T→A); and 3860(C→T).

**Table 1**

| Number | Primer sequence |
|---|---|
| 1 | 5'-ttggcgcgccttaagatatcaacaaactccgggaggcagcgtgatgcggcaacaatcacacggatttcccgtgaa-3' |
| 2 | 5'-catatacctgcacgctgaccacactcactttccctgaaaataatccgctcattcagaccgttcacgggaaatccgtgtga-3' |
| 3 | 5'-ggtcagcgtgcaggtatatgggctatgatgtgcccggcgcttgaggctttctgcctcatgacgtgaaggtggtttgttgc-3' |
| 4 | 5'-cgtggtggttaatgaaaattaacttactacggggctatcttctttctgccacacaacacggcaacaaaccaccttcacgt-3' |
| 5 | 5'-aattttcattaaccaccacgaggcatccctatgtctagtccacatcaggatagcctcttaccgcgctttgcgcaaggaga-3' |
| 6 | 5'-tgagacacacgatcaacacacaccagacaagggaacttcgtggtagtttcatggccttcttctccttgcgcaaagcgcgg-3' |
| 7 | 5'-tgtgttgatcgtgtgtctcacactgttgatattcacttatctgacacgaaaatcgctgtgcgagattcgttacagagacg-3' |
| 8 | 5'-cgcctccaggttgctacttaccggattcgtaagccatgaaagccgccacctccctgtgtccgtctctgtaacgaatctcg-3' |
| 9 | 5'-taagtagcaacctggaggcgggcgcaggcccgccttttcaggactgatgctggtctgactactgaagcgcctttataaag-3' |
| 10 | 5'-cggaattcacaacatcagcaaggagaaaggggctaccggcgaaccagcagcccctttataaaggcgcttcagt-3' |

### Example 5

Construction of vector pRSET-A-DAO with recombinant D-amino acid oxidase Primers were synthesized based on published *Trigonopsis variabilis* D-amino acid oxidase gene (Gonzalez, F. J., Montes, J., Martin, F., Lopez, M. C., Ferminan, E., Catalan, J., Galan, M. A., Dominguez, A. Molecular cloning of TvDAO1, a gene encoding a D-amino acid oxidase from Trigonopsis variabilis and its expression in Saccharomyces cerevisiae and Kluyveromyces lactis. Yeast 13:1399-1408, 1997):
5'-NdeI (incorporation of NdeI restriction site)
5'-TAGGGCTGACATATGGCTAAAATCGTTGTTATTGGTGC-3'
3'-BglII (incorporation of BglII restriction site)
5'-TAGGGCTGAAGATCTCTAAAGGTTTGGACGAGTAAGAGC-3'
*T. variabilis* D-amino acid oxidase gene was synthesized using the above primers and Pfu DNA polymerase (Promega), with plasmid pJL (Yang, Y. L. et al. CN1371999A) as template. Plasmid pJL contains *T. variabilis* FA10 D-amino acid oxidase gene (Li, W. et al., Acta Microbiologica Sinica 31:251-253, 1991). PCR mixture contained 40ng pJL, 0.4µM 5-NdeI, 0.4µM 3'-BglII, 50µM dATP, 50µM dTTP, 50µM dCTP, 50µM dGTP, 20mM Tris-HCI (pH8.8), 10mM KCI, 10mM (NH₄)₂SO₄, 2mM MgSO₄, 0.1% Triton X-100, 2.5U Pfu DNA polymerase. The volume of the mixture was made up to 50µL with sterile deionized water.

PCR profile was as follows:
94°C, 1 min 10 cycles 94°C, 1 min 25 cycles
94°C, 5 min →50°C, 1 min →→→→→ 60°C, 1 min →→→→→ 72°C, 10 min
72°C, 2 min 72°C, 2 min

The PCR product (1,098bp) contains NdeI restriction site at the 5' end and BglII restriction site at the 3' end. The PCR product was resolved in 1% agarose, purified and digested by NdeI and BglII and then ligated with 2.9kb NdeI/BglII restricted pRSETA (Invitrogen), generating pRSET-A-DAO. The plasmid was used to transform competent *E. coli* BL21(DE3)pLysS (Novagen), spread onto LB agar containing ampicillin and incubated at 37°C overnight. Plasmid was extracted in accordance with Molecular Cloning-A Laboratory Manual (Sambrook, J. et al., 1989, CSHL Press) and sequenced. The DNA fragment was confirmed as *T. variabilis* D-amino acid oxidase gene as Sequence 4.

### Example 6

### Construction of expression vector with recombinant D-amino acid oxidase GHA

Recombinant D-amino acid oxidase GHA was constructed by site-directed mutagenesis, which was based on the procedures in PCR Protocols (Ed. John M.S. Bartlett and David Stirling, Totowa, N.J.: Humana Press, 2003).

Primers were synthesized in accordance with the sequence of cloned *T. variabilis* D-amino acid oxidase (Sequence 4):
Primer A
   5'-TAGGGCTGACATATGGCTAAAATCGTTGTTATTG-3'
Primer B
   5'-TAGGGCTGAAGATCTCTAAAGGTTTGGACGAG-3'
Primer C 1
   5'-GCAGGTGCCAACTGGCTCCCGTTTTACGATGGAGGCAAG-3'
Primer D
   5'-GAGCCAGTTGGCACCTGCCCAAGG-3'

Primers A and B are a pair of outer primer. Primer A contains NdeI restriction site, with a portion of nucleotides overlapped with the 5'-end of the D-amino acid oxidase gene; primer B contains BglII restriction site, with a portion of nucleotides overlapped with the 3'-end of the D-amino acid oxidase gene. Primers C 1 and D are inner primers. Primer C1 converts the 53rd amino acid residue of wild-type D-amino acid oxidase from threonine (Thr) to proline (Pro). Primer D contains a portion of nucleotides overlapped with primer C1.

PCR was performed, using pRSET-A-DAO as template, to synthesize fragment 1 (using primers A and D) and fragment 2 (using primers B and C1). PCR mixture contained: 20ng pRSET-A-DAO, 20mM Tris-HCI (pH8.8), 10mM KCI, 10mM (NH₄)₂SO₄, 2mM MgSO₄, 0.1% Triton X-100, 0.4µM primer A and 0.4µM primer D (for synthesizing fragment 1) or 0.4µM primer B and 0.4µM primer C1 (for synthesizing fragment 2), 50µM dATP, 50µM dTTP, 50µM dCTP, 50µM dGTP, 1.5U Pfu DNA polymerase. The volume of the mixture was made up to 50µL with sterile deionized water.

PCR profile was as follows:
94°C, 1 min 30 cycles
94°C, 2 min →53°C, 1 min →→→→→ 72°C, 10 min
72°C, 1 min

The amplified fragment 1 and fragment 2 were resolved in and purified from 1% agarose, and used to generate full-length D-amino acid oxidase GHA gene. PCR mixture for synthesizing the full-length gene contained: 20ng fragment 1, 20ng fragment 2, 20mM Tris-HCl (pH8.8), 10mM KCI, 10mM (NH₄)₂SO₄, 2mM MgSO₄ 0.1% Triton X-100, 0.4µM primer A and 0.4µM primer B, 50µM dATP, 50µM dTTP, 50µM dCTP, 50µM dGTP, 1.5U Pfu DNA polymerase. The volume of the mixture was made up to 50µL with sterile deionized water.

PCR profile was as follows:
94°C, 1 min 35 cycles
94°C, 2 min →53°C, 1 min →→→→→ 72°C, 10 min
72°C, 2 min

The full-length recombinant D-amino acid oxidase GHA gene fragment was obtained, cut with NdeI and BglII and ligated with pRSET-kan, generating pRSET-kan-DAOGHA. The plasmid was used to transform competent *E*. *coli* BL21(DE3)pLysS (Novagen), spread onto LB agar containing kanamycin and incubated at 37°C overnight. Plasmid was extracted, and the insert was sequenced and confirmed as recombinant D-amino acid oxidase GHA as Sequence 5.

### Example 7

### Construction of vector pHS-GHA (Fig. 1)

Vector pRSET-kan-DAOGHA was cut by NdeI and BglII to release a DNA fragment (1,074bp, containing D-amino acid oxidase GHA) and resolved in 0.8% agarose, purified and ligated to the large fragment of NdeI/BglII-digested pRSET-lac-GI-hok/sok-kan, generating pHS-GHA. The plasmid was used to transform competent E. *coli* BL21(DE3)pLysS (Novagen), generating clone BL-HS-GHA, spread onto LB agar containing 50µg/mL kanamycin and incubated at 37°C overnight. Plasmid was extracted in accordance with Molecular Cloning-A Laboratory Manual (Sambrook, J. et al., 1989, CSHL Press) and sequenced (Sequence 1). Nucleotide variations were observed in a number of positions: 1390 (C→G); 1535 (missed a T); 1826(A→T); 1848(C→T); 2501(G→T); 2577(T→A); and 3882(C→T).

### Example 8

### Construction of vector pT7-kan-GHA

Vector pRSET-kan-DAOGHA was cut by NdeI and BglII to release a DNA fragment (1,074bp, containing D-amino acid oxidase GHA) and resolved in 0.8% agarose, purified and ligated to NdeI/BglII-digested pRSET-kan, generating pT7-kan-GHA. The plasmid was used to transform competent E. *coli* BL21(DE3)pLysS (Novagen), generating clone BL-T7K-GHA, spread onto LB agar containing 50µg/mL kanamycin and incubated at 37°C overnight.

### Example 9

### Construction of vector pT7-amp-GHA

Vector pRSET-kan-DAOGHA was cut by NdeI and BglII to release a DNA fragment (1,074bp, containing D-amino acid oxidase GHA) and resolved in 0.8% agarose, purified and ligated to NdeI/BgIII-digested pRSET-A, generating pT7-amp-GHA. The plasmid was used to transform competent E. *coli* BL21(DE3)pLysS (Novagen), generating clone BL-T7A-GHA, spread onto LB agar containing 100µg/mL ampicillin and incubated at 37°C overnight.

### Example 10

### Construction of vector pT7-kan-ACY (Fig. 2)

The following primers were synthesized based on the sequence of known glutaryl-7-ACA acylase gene of *Pseudomonas* sp. SE83 (Matsuda, A. et al., 1987, J. Bacteriol. 169, 5821-5826).
NdeI-ACY
5'-CATATGAACGCTCCCGTCCCCGTCCC-3'
(NdeI restriction site is underlined);
BgIII-ACY
5'-AGATCTTCAGATGGTGAAGCGGGCAC-3'
(BglII restriction site is underlined).

PCR was performed using *Pseudomonas* sp. SE83 DNA as template to generate a 1,676bp PCR product. PCR mixture contained 50ng *Pseudomonas* sp. SE83 DNA, 0.4µM NdeI-ACY, 0.4µM BglII-ACY, 50µM dATP, 50µM dTTP, 50µM dCTP, 50µM dGTP, 20mM Tris-HCl (pH8.8), 10mM KCI, 10mM (NH₄)₂SO₄, 2mM MgSO₄, 0.1% Triton X-100, 2.5U Pfu DNA polymerase (Promega). The volume of the mixture was made up to 50µL with sterile deionized water.

PCR profile was as follows:
94°C, 1 min 35 cycles
95°C, 5 min →50°C, 1 min →→→→→ 72°C, 10 min
72°C, 3 min

The PCR product (1,676bp) contains NdeI restriction site at the 5' end and BglII restriction site at the 3' end. The PCR product was resolved in 0.8% agarose, cut with NdeI and BglII and ligated to NdeI/BglII-digested pRSET-kan, generating pT7-kan-ACY. The plasmid was used to transform competent E. *coli* BL21(DE3)pLysS (Novagen), generating clone BL-T7K-ACY, spread onto LB agar containing 50µg/mL kanamycin and incubated at 37°C overnight. Plasmid was extracted in accordance with Molecular Cloning-A Laboratory Manual (Sambrook, J. et al., 1989, CSHL Press) and sequenced (Sequence 2). Nucleotide variations were observed in a number of positions: 2260 (G→T); 2336 (T→A); 3641(C→T); and 4117(G→C).

### Example 11

### Construction of vector pT7-amp-ACY

A PCR product containing glutaryl-7-ACA acylase gene of *Pseudomonas* sp. SE83 was synthesized in accordance with Example 10. The PCR product was resolved in 0.8% agarose, cut with NdeI and BglII, and ligated to NdeI/BglII-digested pRSET-A, generating pT7-amp-ACY. The plasmid was used to transform competent *E*. *coli* BL21(DE3)pLysS (Novagen), generating clone BL-T7A-ACY, spread onto LB agar containing 100µg/mL ampicillin and incubated at 37°C overnight.

### Example 12

### Examination of plasmid stability of vector pHS-GHA

A single colony of clone BL-HS-GHA (Example 7) was picked from LB agar containing 50µg/mL kanamycin and grown in 5mL LB broth containing 50µg/mL kanamycin at 37°C for 12 hours (shaking at 200rpm). An aliquot of 500µL of culture was inoculated to 50mL corn steep liquor medium, incubated at 35°C for 24 hours (shaking at 200rpm).

### Preparation of corn steep liquor 1:

Dissolved 300g corn steep liquor (purchased from North China Pharmaceutical Kangxin Co., Ltd.) in 300mL distilled water and then centrifuged (5,000g, 8min.) to harvest the supernatant as corn steep liquor 1. The pellet was kept for later use.

### Preparation of corn steep liquor 2:

Dissolved the above mentioned pellet in 600mL distilled water and then centrifuged (5,000g, 8min.) to harvest the supernatant as corn steep liquor 2.

A 50mL corn steep liquor medium contained:

| | |
|---|---|
| Corn steep liquor 1 | 4mL |
| Corn steep liquor 2 | 4mL |
| Yeast extract | 0.2g |
| Ammonium sulphate | 0.075g |
| Disodium hydrogen phosphate | 0.25g |
| Potassium dihydrogen phosphate | 0.04g |
| Sodium chloride | 0.075g |

They were dissolved in 50mL distilled water, adjusted to pH 7.15 by 10N sodium hydroxide, and sterilized by autoclaving, and 0.0025g kanamycin was added. After 24 hours incubation, culture sample was removed and diluted by 10⁸ times and spread onto LB agar plates and incubated at 37°C overnight. Colonies were imprinted onto LB agar plates and LB agar containing 50µg/mL kanamycin by toothpicks and incubated at 37°C overnight. The number of colonies on each plate was counted (Table 2).

### Example 13

### Examination of plasmid stability of vector pT7-kan-GHA

A single colony of clone BL-T7K-GHA (Example 8) was picked from LB agar containing 50µg/mL kanamycin and grown in 5mL LB broth containing 50µg/mL kanamycin at 37°C for 12 hours (shaking at 200rpm). An aliquot of 500µL of culture was inoculated to 50mL corn steep liquor medium, incubated at 35°C for 24 hours (shaking at 200rpm).

### Preparation of corn steep liquor 1:

Dissolved 300g corn steep liquor (purchased from North China Pharmaceutical Kangxin Co., Ltd.) in 300mL distilled water and then centrifuged (5,000g, 8min.) to harvest the supernatant as corn steep liquor 1. The pellet was kept for later use.

### Preparation of corn steep liquor 2:

Dissolved the above mentioned pellet in 600mL distilled water and then centrifuged (5,000g, 8min.) to collect the supernatant as corn steep liquor 2.

A 50mL corn steep liquor medium contained:

| | |
|---|---|
| Corn steep liquor 1 | 4mL |
| Corn steep liquor 2 | 4mL |
| Yeast extract | 0.2g |
| Ammonium sulphate | 0.075g |
| Disodium hydrogen phosphate | 0.25g |
| Potassium dihydrogen phosphate | 0.04g |
| Sodium chloride | 0.075g |

They were dissolved in 50mL distilled water, adjusted to pH 7.15 by 10N sodium hydroxide, and sterilized by autoclaving, and 0.0025g kanamycin was added. After 24 hours incubation, culture sample was removed and diluted by 10⁸ times and spread onto LB agar plates and incubated at 37°C overnight. Colonies were imprinted onto LB agar plates and LB agar containing 50µg/mL kanamycin by toothpicks and incubated at 37°C overnight. The number of colonies on each plate was counted (Table 2).

### Example 14

### Examination of plasmid stability of vector pT7-amp-GHA

A single colony of clone BL-T7A-GHA (Example 9) was picked from LB agar containing 100µg/mL ampicillin and grown in 5mL LB broth containing 100µg/mL ampicillin at 37°C for 12 hours (shaking at 200rpm). An aliquot of 500µL of culture was inoculated to 50mL corn steep liquor medium, incubated at 35°C for 24 hours (shaking at 200rpm).

A 50mL corn steep liquor medium contained:

| | |
|---|---|
| Corn steep liquor 1 | 4mL |
| Corn steep liquor 2 | 4mL |
| Yeast extract | 0.2g |
| Ammonium sulphate | 0.075g |
| Disodium hydrogen phosphate | 0.25g |
| Potassium dihydrogen phosphate | 0.04g |
| Sodium chloride | 0.075g |

They were dissolved in 50mL distilled water, adjusted to pH 7.15 by 10N sodium hydroxide, and sterilized by autoclaving, and 0.0025g kanamycin was added.

After 24 hours incubation, culture sample was removed and diluted by 10⁸ times and spread onto LB agar plates and incubated at 37°C overnight. Colonies were imprinted onto LB agar plates and LB agar containing 100µg/mL ampicillin by toothpicks and incubated at 37°C overnight. The number of colonies on each plate was counted (Table 2).

As shown in Table 2, vectors containing kanamycin resistance gene (pHS-GHA and pT7-kan-GHA) are more stable than vector containing ampicillin resistance gene (pT7-amp-GHA). After 24 hours, vector in clone BL-T7A-GHA (pT7-amp-GHA) was almost lost completely. The reason was attributed to the different resistance mechanisms of the two resistance genes: the gene product of ampicillin resistance gene is β-lactamase, which secretes into periplasm (the space between cell wall and cell membrane) and hydrolyzes ampicillin in culture medium. As a result, the level of ampicillin in the culture medium drops rapidly. The disappearance of selective pressure of ampicillin lead to the lost of E. *coli* (with ampicillin resistance gene) in culture medium. The gene product of kanamycin resistance gene is kanamycin phosphotransferase, which does not destroy selective pressure. In addition, the hok/sok DNA fragment in vector pHS-GHA further enhanced the stability of the vector in E. *coli* and as much as 85.59% of the cell consequently contained the vector..

**Table 2**

| Clone | Expression vector | Number of colonies on LB agar plate | Number of colonies on LB agar plate containing ampicillin/kanamycin gene | Percentage |
|---|---|---|---|---|
| BL-HS-GHA | pHS-GHA | 576 | 493 | 85.59 |
| BL-T7K-GHA | pT7-kan-GHA | 576 | 5 | 0.87 |
| BL-T7A-GHA | pT7-amp-GHA | 576 | 1 | 0.17 |

### Example 15

### Fermentation of clone BL-T7K-ACY

A single colony of clone BL-T7K-ACY (Example 10) was picked from LB agar containing 50µg/mL kanamycin and grown in 5mL LB broth containing 50µg/mL kanamycin at 37°C for 12 hours (shaking at 200rpm). An aliquot of 500µL of culture was inoculated to 50mL corn steep liquor medium, incubated at 30°C for 24 hours (shaking at 200rpm).

A 50mL medium contained:

| | |
|---|---|
| Yeast extract | 0.35g |
| Disodium hydrogen phosphate | 0.35g |
| Potassium dihydrogen phosphate | 0.35g |
| Dipotassium hydrogen phosphate | 0.48g |
| Ammonium sulphate | 0.06g |
| Ammonium chloride | 0.01g |
| Glycerol | 0.5mL |
| Calcium chloride | 0.00055g |

The chemicals were dissolved in 50mL distilled water and sterilized by autoclaving and 0.0025g kanamycin was added.

### Example 16

### Fermentation of clone BL-T7A-ACY

A single colony of clone BL-T7A-ACY (Example 11) was picked from LB agar containing 50µg/mL kanamycin and grown in 5mL LB broth containing 100µg/mL ampicillin at 37°C for 12 hours (shaking at 200rpm). An aliquot of 500µL of culture was inoculated to 50mL corn steep liquor medium, incubated at 30°C for 24 hours (shaking at 200rpm).

A 50mL medium contained:

| | |
|---|---|
| Yeast extract | 0.35g |
| Disodium hydrogen phosphate | 0.35g |
| Potassium dihydrogen phosphate | 0.35g |
| Dipotassium hydrogen phosphate | 0.48g |
| Ammonium sulphate | 0.06g |
| Ammonium chloride | 0.01g |
| Glycerol | 0.5mL |
| Calcium chloride | 0.00055g |

The chemicals were dissolved in 50mL distilled water and sterilized by autoclaving and 0.0025g ampicillin was added.

### Example 17

### Partial purification of D-amino acid oxidase GHA and Pseudomonas sp. SE83 glutaryl-7-ACA acylase

Fermentation was performed as in Examples 12-16. Culture medium (50mL) was centrifuged at 4°C (8,000g, 8min.). Supernatant was discarded and cell pellet was resuspended in sodium phosphate buffer. For clones BL-HS-GHA, BL-T7K-GHA and BL-T7A-GHA, the pellets were resuspended in 50mL sodium phosphate buffer (50mM, pH7.5); for clones BL-T7K-ACY and BL-T7A-ACY, the pellets were resuspended in 10mL sodium phosphate buffer (50mM, pH8). The cells were lysed by 4 cycles of freeze-thaw, deep freezing by liquid nitrogen and thawing at 50°C in water bath and were used as partial purified enzymes.

### Example 18

### Degradation of CPC/glutaryl-7-ACA by D-amino acid oxidase GHA from clones BL-HS-GHA, BL-T7K-GHA and BL-T7A-GHA

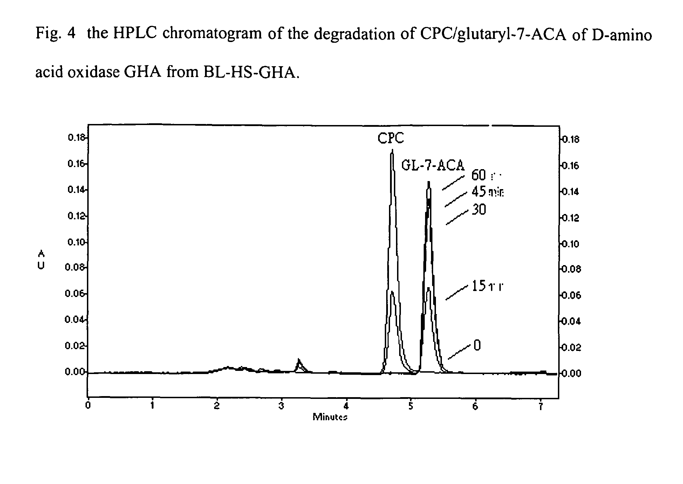

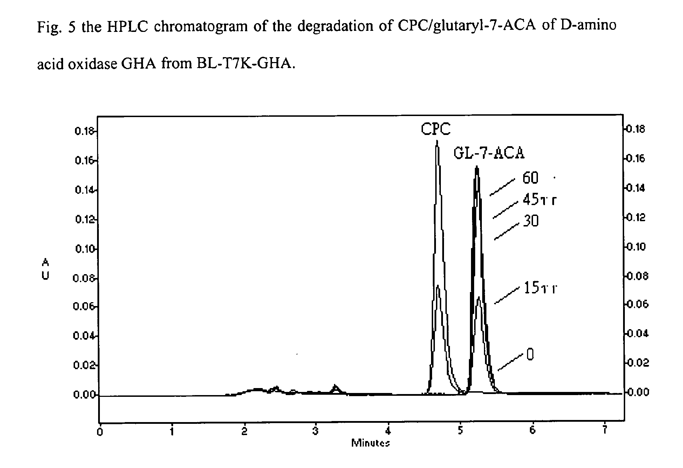

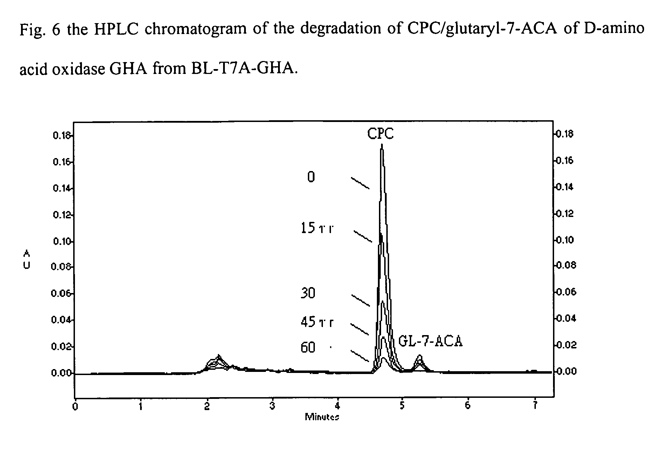

The procedures were performed in accordance with Isogai, T., et al. J. Biochem. [Tokyo] 108:1063-1069, 1990, with modifications. Reaction mixture contained 2mL 150mM CPC sodium and 2mL partially purified D-amino acid oxidase GHA (Example 17) at 37°C with continuously stirring (450rpm) and the pH value was maintained at 7.5 by 5N sodium hydroxide. Aliquots (20µL) were withdrawn at different time points (0, 15, 30, 45, 60 min., Figs. 4, 5 and 6), mixed with 2µL 3% hydrogen peroxide. The mixture was centrifuged (10,000g, 10sec.) and 10µL of supernatant was mixed with 990µL HPLC mobile phase (50mM potassium phosphate, pH7; 5% acetonitrile), then analyzed by HPLC. HPLC column: Diamonsil™ C18, 250 x 4.6mm (Diam Company, Beijing); column temperature: 30°C; flow rate: 1mL/min; scanning: 260nm UV. When comparing among Figs. 4, 5 and 6, D-amino acid oxidase GHA produced from clones BL-HS-GHA and BL-T7K-GHA did not cause degradation to CPC/glutaryl-7-ACA; on the contrary, D-amino acid oxidase GHA produced from clone BL-T7A-GHA significantly degraded CPC/glutaryl-7-ACA, thus significantly reducing the production level of glutaryl-7-ACA.

### Example 19

### Degradation of glutaryl-7-ACA/7-ACA by glutaryl-7-ACA acylase of Pseudomonas sp. SE83 from clones BL-T7K-ACY and BL-T7A-ACY

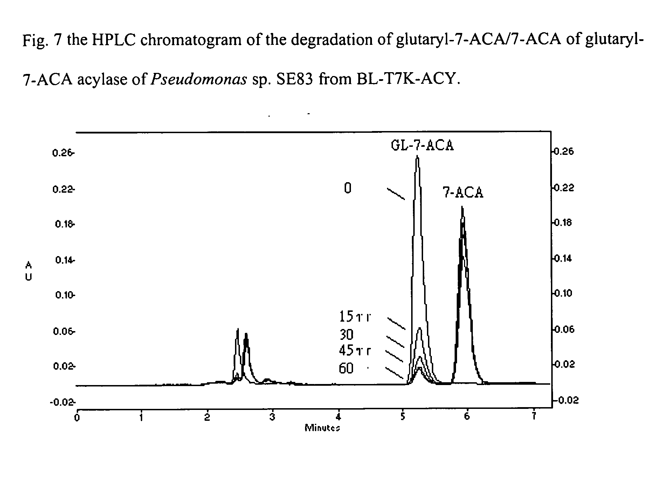

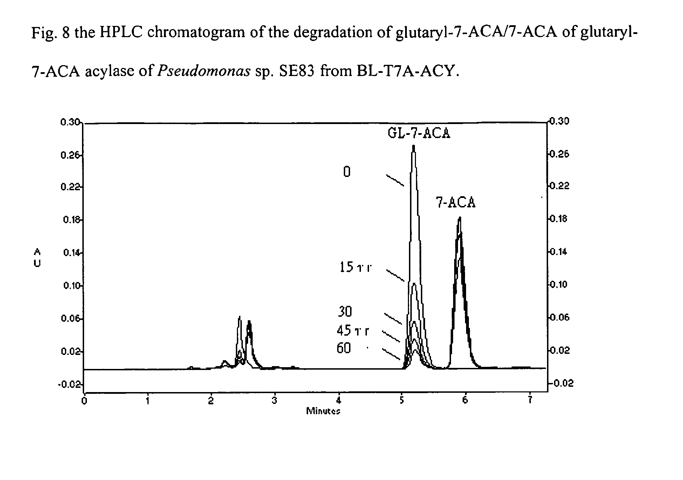

The procedures were performed in accordance with Binder, R. et al., 1994, Appl. Environ. Microbiol. 60, 1805-1809, with modifications. Reaction mixture contained 10mL partially purified glutaryl-7-ACA acylase of *Pseudomonas* sp. SE83 (Example 17) and 10mL 150mM glutaryl-7-ACA (prepared as described in Shibuya, Y. et al., 1981, Agric. Biol. Chem. 45, 1561-1567) at 37°C with continuously stirring (450rpm) and the pH value was maintained at 8 by 5N sodium hydroxide. Aliquots (20µL) were withdrawn at different time points (0, 15, 30, 45, 60 min., Figs. 7 and 8) and centrifuged (10,000g, 10sec.) and 10µL of supernatant was mixed with 990µL HPLC mobile phase (50mM potassium phosphate, pH7; 5% acetonitrile), then analyzed by HPLC. HPLC conditions were identical to that in Example 18. When comparing between Figs. 7 and 8, glutaryl-7-ACA acylase of *Pseudomonas* sp. SE83 produced from clone BL-T7K-ACY did not cause degradation to glutaryl-7-ACA/7-ACA (60 min. sample, the amount of 7-ACA synthesized was 0.178mg/mL); on the contrary, glutaryl-7-ACA acylase of *Pseudomonas* sp. SE83 produced from clone BL-T7A-ACY degraded glutaryl-7-ACA/7-ACA (60 min. sample, the amount of 7-ACA synthesized was 0.145mg/mL), thus reducing the production level of 7-ACA by about 19%.

This invention is not limited by the detailed description provided in the Examples above. Various modifications can be made by those skilled in the field and these modifications should be regarded as within the scope of the claims of the invention.

## Claims

1. An *Escherichia coli* expression vector that contains a promoter to drive the expression of a target protein; said expression vector is constructed from vector pRSET-A; wherein the ampicillin resistance gene is replaced by a kanamycin resistance gene.

2. The expression vector according to claim 1, wherein the promoter of said expression vector is lac promoter.

3. The expression vector according to claim 2, wherein said expression vector contains expressible hok/sok genes.

4. The expression vector according to claim 3, wherein said expression vector contains ribosome binding site AGAAGGA.

5. The expression vector according to any of claims 1-4, wherein the target protein to be expressed is D-amino acid oxidase.

6. The expression vector according to claim 4, wherein said D-amino acid oxidase is *Trigonopsis variabilis* D-amino acid oxidase GHA.

7. The expression vector according to claim 6, which is pHS-GHA, with the DNA sequence of Sequence 1.

8. The expression vector according to claim 1, wherein the target protein to be expressed is glutaryl-7-ACA acylase.

9. The expression vector according to claim 8, wherein said glutaryl-7-ACA acylase is glutaryl-7-ACA acylase of *Pseudomonas* sp. SE83.

10. The expression vector according to claim 9, which is pT7-kan-ACY, with the DNA sequence of Sequence 2.

11. A method to express a target protein in E. *coli,* and said *E. coli* is transformed by a expression vector containing the target protein; said target protein is controlled by promoter; the method comprises the growth of E. *coli* in culture medium for production of the target protein, preferably the expression vector is constructed from vector pRSET-A, wherein the ampicillin resistance gene is replaced by a kanamycin resistance gene.

12. The method according to claim 11, said target protein is either D-amino acid oxidase gene or glutaryl-7-ACA acylase gene.

13. The method according to claims 11 and 12, said E. *coli* is grown in culture medium containing 50-100µg/mL kanamycin.
